# EUROPEAN PATENT APPLICATION

(11) **EP 2 255 718 A1**
(43) Date of publication of application: **01.12.2010**
(21) Application number: 09725000.5
(22) Date of filing: 16.03.2009
(51) Int. Cl.: A61B 1/00, A61B 5/06, A61B 5/07

(54) **CAPSULE MEDICAL DEVICE**

(30) Priority: 24.03.2008 JP 2008076338
(71) Applicant: Olympus Corporation, Shibuya-ku Tokyo 151-0072 (JP)
(72) Inventor: ISHIHARA, Yasushige, Tokyo 151-0072 (JP)
(74) Representative: Gunzelmann, Rainer
(86) International application number: PCT/JP2009/055005
(87) International publication number: WO 2009/119363

(57) **Abstract**

A capsule medical device capable of alleviating a burden on a patient is provided. Provided is a capsule medical device (1) that includes a casing (2) having a capsule shape; a measurement-information acquiring section (4, 6), disposed in the casing (2), for acquiring measurement information from an examination site positioned outside the casing (2); a locating-information acquiring section (5) for acquiring information for locating the examination site from which the measurement information has been acquired by the measurement-information acquiring section (4, 6); and a storage section (7) for storing the measurement information acquired by the measurement-information acquiring section (4, 6) in association with the information for locating the examination site, acquired by the locating-information acquiring section (5).

## Description

### Technical Field

The present invention relates to capsule medical devices.

### Background Art

In the related art, there is a known capsule medical device that has a casing having a capsule shape, that is introduced into a patient's body, and that takes an image of the interior of a body cavity to acquire image information (see, for example, Patent Citation 1.).
This capsule medical device includes wireless transmission means to transmit the acquired image information toward an external device disposed outside the patient's body.

Patent Citation 1:
Japanese Unexamined Patent Application, Publication No. 2003-70728

### Disclosure of Invention

One problem with the capsule medical device of Patent Citation 1, however, is that a great burden is placed on the patient because they must have an examination by wearing a portable external device or staying near a stationary external device, which is restrictive.

An object of the present invention, which has been made in light of the circumstances described above, is to provide a capsule medical device capable of alleviating the burden on the patient.

To achieve the above object, the present invention provides the following solutions.
Provided is a capsule medical device that includes a casing having a capsule shape; a measurement-information acquiring section, disposed in the casing, for acquiring measurement information from an examination site positioned outside the casing; a locating-information acquiring section for acquiring information for locating the examination site from which the measurement information has been acquired by the measurement-information acquiring section; and a storage section for storing the measurement information acquired by the measurement-information acquiring section in association with the information for locating the examination site, acquired by the locating-information acquiring section.

According to the present invention, when the casing having a capsule shape is introduced into a patient's body cavity, the measurement-information acquiring section operates to acquire measurement information from the examination site outside the casing, and the locating-information acquiring section operates to acquire information for locating the examination site. The measurement information thus acquired is stored in the storage section in association with the information for locating the examination site.

Thus, because the measurement information is stored in the storage section, it does not need to be transmitted and accumulated outside the body and no external device needs to be attached or installed, so that the burden on the patient can be alleviated and battery consumption can be reduced, allowing examination to be carried out over an extended period of time. In addition, because the stored measurement information is associated with the information for locating the examination site, it can be easily checked later to which examination site the particular measurement information corresponds.

In the above invention, the measurement-information acquiring section may be an image-acquisition device for acquiring image information by taking an image of the examination site outside the casing.
By doing so, the condition of the examination site can be examined in greater detail on the basis of image information.

In the above invention, additionally, the measurement-information acquiring section may calculate a maximum or average value of luminance values of individual pixels acquired by the image-acquisition device as the measurement information for the examination site.
By calculating the maximum value of the luminance values of the individual pixels, the amount of fluorescent agent or autofluorescent substance that accumulates specifically in a diseased area such as cancer tissue can be measured to check the presence of a diseased area. On the other hand, by calculating the average value of the luminance values of the individual pixels, noise can be reduced to improve the reliability of examination.

In the above invention, additionally, the measurement-information acquiring section may calculate the number of pixels whose luminance values acquired by the image-acquisition device exceed a predetermined threshold as the measurement information for the examination site.
By doing so, the area of a region where a certain amount of fluorescent agent or autofluorescent substance that accumulates specifically in a diseased area such as cancer tissue is present can be measured, thus improving the diagnostic accuracy of a region that is possibly a diseased area.

In the above invention, additionally, the measurement-information acquiring section may be a light detector for detecting brightness information at the examination site outside the casing.
By doing so, the amount of data stored in the storage section can be reduced to facilitate examination over an extended period of time.

In the above invention, additionally, the locating-information acquiring section may be a timer for measuring examination time.
By doing so, the examination time can be measured using the timer to allow an examination site, such as an organ, shown in image information to be easily identified later.

In the above invention, additionally, the locating-information acquiring section may be a pressure sensor for detecting a pressure exerted on the casing.
By doing so, changes in the pressure exerted on the casing can be monitored using the pressure sensor to locate the examination site on the basis of the pressure value detected. For example, the examination site can be determined to be a narrow organ such as the esophagus, the small intestine, or the large intestine if the pressure received by the casing is large, and can be determined to be a large organ such as the stomach if the pressure is small.

In the above invention, additionally, the locating-information acquiring section may be an enterobacterium sensor for detecting enterobacteria.
By doing so, it can be easily determined that the examination site has shifted to the large intestine if the enterobacterium sensor detects enterobacteria.

In the above invention, additionally, the locating-information acquiring section may be a pH sensor for detecting a pH value around the casing.
By doing so, it can be easily determined on the basis of the output of the pH sensor that the examination site has shifted to the stomach because the stomach is lower in pH value than other organs.

The present invention provides the advantage of alleviating the burden on the patient and easily identifying the examination site later.

### Brief Description of Drawings

[FIG. 1] Fig. 1 is a longitudinal sectional view showing a capsule medical device according to an embodiment of the present invention.
[FIG. 2] Fig. 2 is a block diagram showing the internal structure of the capsule medical device in Fig. 1.
[FIG. 3] Fig. 3 is a graph showing the relationship between fluorescence intensity and elapsed time, acquired by the capsule medical device in Fig. 1.
[FIG. 4] Fig. 4 is a partial longitudinal sectional view showing a first modification of a locating-information acquiring section of the capsule medical device in Fig. 1.
[FIG. 5] Fig. 5 is a block diagram showing the internal structure of the capsule medical device in Fig. 4.
[FIG. 6] Fig. 6 is a graph showing the relationship between pressure values and elapsed time, acquired by the locating-information acquiring section in Fig. 4.
[FIG. 7] Fig. 7 is a partial longitudinal sectional view showing a second modification of a locating-information acquiring section of the capsule medical device in Fig. 1.
[FIG. 8] Fig. 8 is a block diagram showing the internal structure of the capsule medical device in Fig. 7.
[FIG. 9] Fig. 9 is a graph showing the relationship between pressure values and elapsed time, acquired by the locating-information acquiring section in Fig. 7.
[FIG. 10] Fig. 10 is a block diagram showing the internal structure of a third modification of a locating-information acquiring section of the capsule medical device in Fig. 1.
[FIG. 11] Fig. 11 is a graph showing the relationship between pH values and elapsed time, acquired by the locating-information acquiring section in Fig. 10.
[FIG. 12] Fig. 12 is a partial longitudinal sectional view showing a fourth modification of a locating-information acquiring section of the capsule medical device in Fig. 1.
[FIG. 13] Fig. 13 is a graph showing the relationship between pressure values and elapsed time, acquired by the locating-information acquiring section in Fig. 12.
[FIG. 14] Fig. 14 is a graph showing temporal changes in luminance information and area information, acquired by a fifth modification of the capsule medical device in Fig. 1.

### Explanation of Reference:

- 1:: capsule medical device
- 2:: casing
- 4:: light-detecting section (measurement-information acquiring section)
- 4b:: photodetector (light detector)
- 5:: timer (locating-information acquiring section)
- 6:: information-processing section (measurement-information acquiring section)
- 7:: memory (storage section)
- 9:: enterobacterium sensor (locating-information acquiring section)
- 14:: pH sensor (locating-information acquiring section)

### Best Mode for Carrying Out the Invention

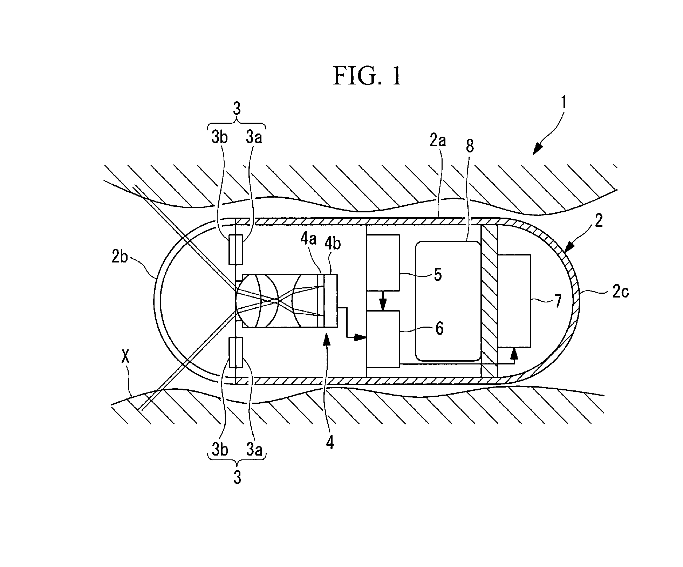

A capsule medical device 1 according to an embodiment of the present invention will be described below with reference to Figs. 1 to 3.
As shown in Fig. 1, the capsule medical device 1 according to this embodiment includes a casing 2 having a capsule shape in which the two ends of a cylindrical casing body 2a are sealed by a hemispherical transparent window 2b and end plate 2c, an illumination section 3 accommodated in the casing 2 and emitting excitation light through the transparent window 2b, a light-detecting section (measurement-information acquiring section) 4 for detecting fluorescence entering the casing 2 through the transparent window 2b from an examination site outside the casing 2, a timer (locating-information acquiring section) 5 for measuring the time elapsed from the introduction of the capsule medical device 1 into a body cavity, an information-processing section (measurement-information acquiring section) 6 for associating the fluorescence intensity detected by the light-detecting section 4 with the elapsed time measured by the timer 5, a memory (storage section) 7 for storing the information associated by the information-processing section 6, and a battery 8 for supplying power to the individual sections. Wiring from the battery 8 to the individual sections is not shown.

The illumination section 3 includes, for example, LEDs 3a for emitting wideband light and excitation light filters 3b for transmitting, of the light emitted from the LEDs 3a, only light at the excitation wavelength, so that it exits through the transparent window 2b.
The light-detecting section 4 includes an excitation light cut filter 4a for blocking excitation light and a photodetector (light detector) 4b for detecting the intensity of fluorescence from which the excitation light is removed.

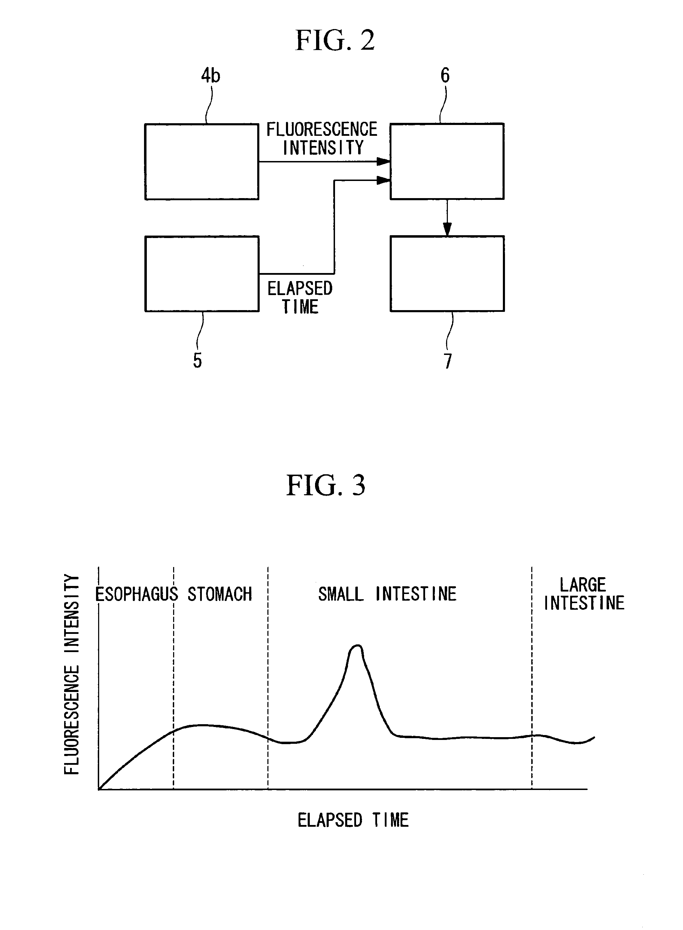

As shown in Fig. 2, the information-processing section 6 is connected to the photodetector 4b and the timer 5 to output, to the memory 7, fluorescence intensity information transmitted from the photodetector 4b in association with the elapsed time transmitted from the timer 5.

The operation of the thus-configured capsule medical device 1 according to this embodiment will now be described.
When the capsule medical device 1 according to this embodiment is introduced into a patient's body cavity X, the timer 5 starts measuring elapsed time. With the capsule medical device 1 disposed in the body cavity X, the illumination section 3 operates to irradiate an examination site with excitation light through the transparent window 2b to excite a fluorescent substance present at the examination site so that it emits fluorescence, which then enters the casing 2 through the transparent window 2b.
The fluorescence emitted from the fluorescent substance is, for example, fluorescence emitted from a dye introduced into the patient by, for example, spraying, intravenous injection, or oral administration in advance before the capsule medical device 1 is administered to the patient and accumulated at the examination site, or autofluorescence emitted from the examination site itself.

The intensity of the fluorescence entering the casing 2 is detected by the light-detecting section 4. Then, the fluorescence intensity information detected by the light-detecting section 4 and the elapsed time measured by the timer 5 at the time of detection of the intensity information are input to the information-processing section 6, where they are output to and stored in the memory 7 in association with each other.

In this way, the capsule medical device 1 according to this embodiment stores the fluorescence intensity information, serving as measurement information for the patient's examination site, in association with the elapsed time indicating the time of measurement so that it can present temporal changes in fluorescence intensity information, as shown in Fig. 3, after the completion of the examination and the recovery of the capsule medical device 1. Because the measurement of elapsed time is started when the capsule medical device 1 is introduced into the body cavity X, for example, as shown in Fig. 3, the position of the examination site at the time of detection of fluorescence intensity information can be roughly shown.

As a result, for instance, in the example shown in Fig. 3, it is possible to assume that a diseased area, such as cancer tissue, is present in the small intestine because an examination site with relatively high fluorescence intensity is present at a position that is assumed, on the basis of elapsed time, to be the position of the small intestine.
In addition, since fluorescence intensity information is used as the measurement information for the patient's examination site in this embodiment, a large amount of information can be stored in the memory 7 because the amount of data thereof is smaller than that of image information. Accordingly, a more detailed examination can be carried out by setting a shorter sampling time for data acquisition.

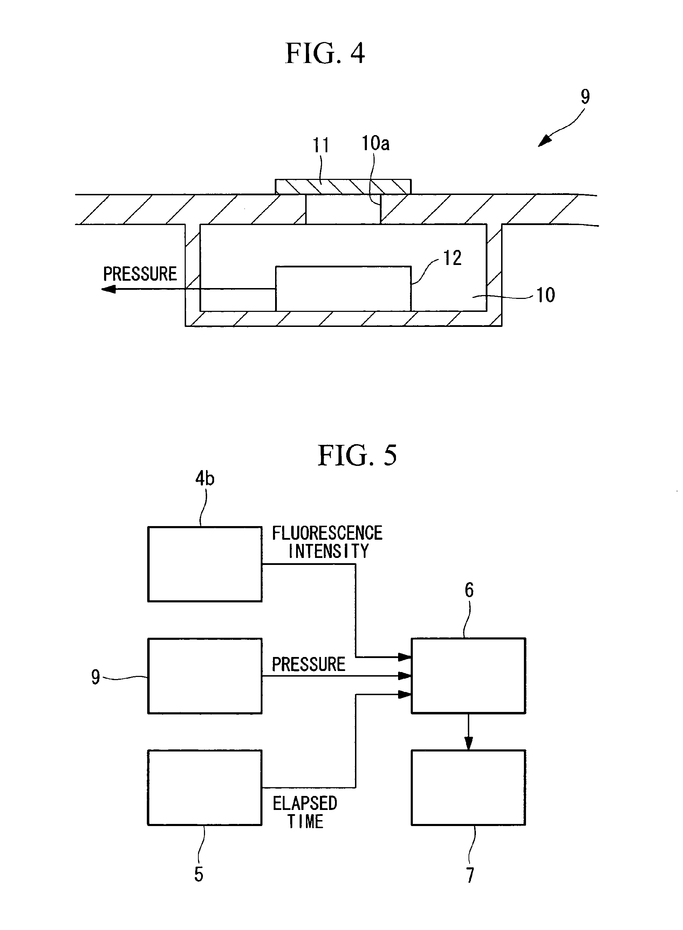

In this embodiment, the elapsed time whose measurement is started when the capsule medical device 1 is introduced into the body cavity X is acquired by the timer 5 as the information for locating the examination site; instead of or in addition to this, the following information may be acquired.
First, as shown in Figs. 4 and 5, an enterobacterium sensor 9 may be used to determine that the capsule medical device 1 has moved to the large intestine at the time of detection of enterobacteria.

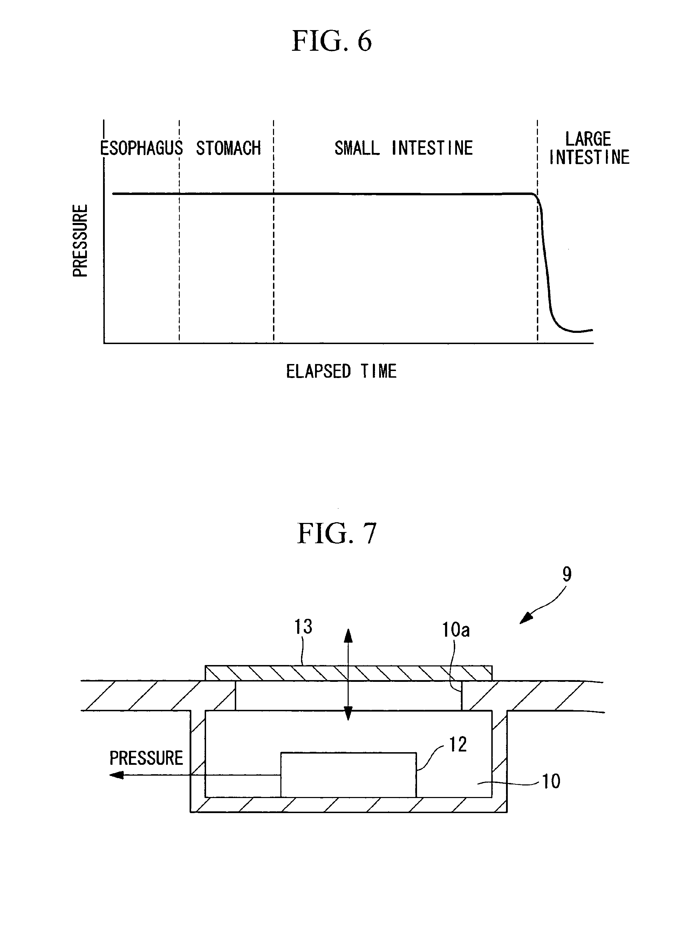

The enterobacterium sensor 9 includes, for example, a pressure chamber 10 set to a predetermined pressure higher than the atmospheric pressure, an azo polymer film 11 that is disposed so as to seal off an opening 10a and that is ruptured by an enzyme secreted by enterobacteria, and a pressure sensor 12 that detects the internal pressure of the pressure chamber 10. Because the internal pressure of the pressure chamber 10 drops as the azo polymer film 11 is ruptured, as shown in Fig. 6, the pressure drop can be detected by the pressure sensor 12 to determine that the examination site has shifted to the large intestine. Since there are individual differences in the time required for the capsule medical device 1 to arrive at the large intestine from the stomach, this method can more accurately locate the examination site because it can directly detect the arrival at the large intestine.

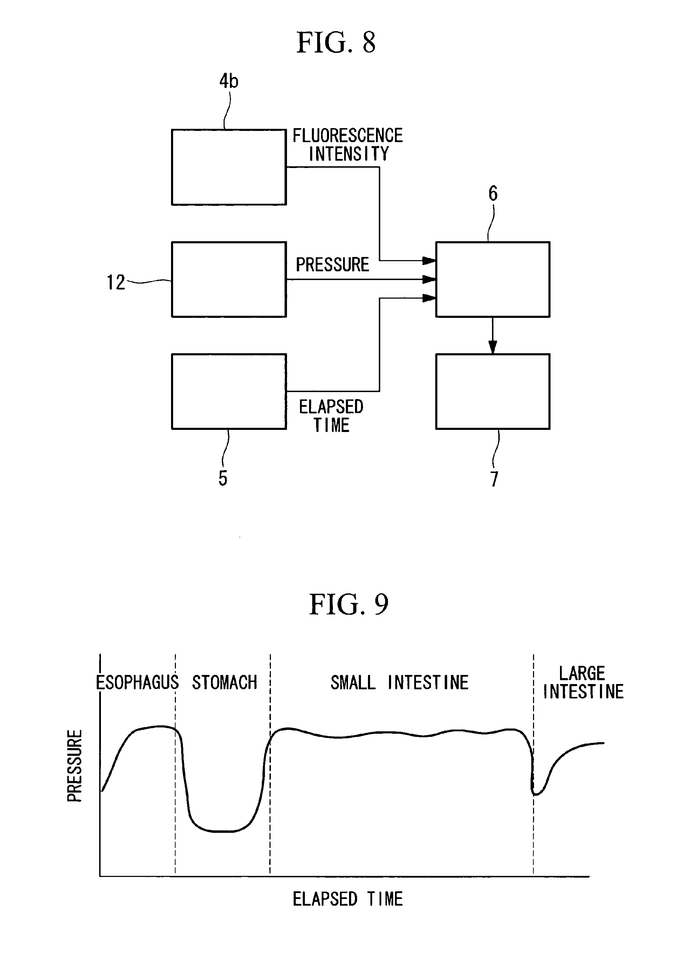

Second, as shown in Figs. 7 and 8, the examination sites corresponding to the fluorescence intensity information may be located on the basis of pressure variations measured using the pressure sensor 12. For example, as shown in Fig. 7, with the opening 10a of the pressure chamber 10 sealed off with an elastic film 13, elastic deformation of the elastic film 13 may be detected as variations in the internal pressure of the pressure chamber 10.

As shown in Fig. 9, the examination site can be determined to be a narrow organ such as the esophagus, the small intestine, or the large intestine if the pressure received from the wall surface of the body cavity X is large, and can be determined to be a large organ such as the stomach if the pressure is small. In addition, the examination site can be located on the basis of the fact that the pressure detected drops temporarily immediately after the passage through the ileocecal valve between the small intestine and the large intestine.

Because pressure values and examination sites cannot be uniquely associated if the pressure sensor 12 is used alone, it is necessary to use the elapsed time measured by the timer 5 in combination therewith, as shown in Figs. 5 and 8, or to store the acquired pressure values in chronological order.

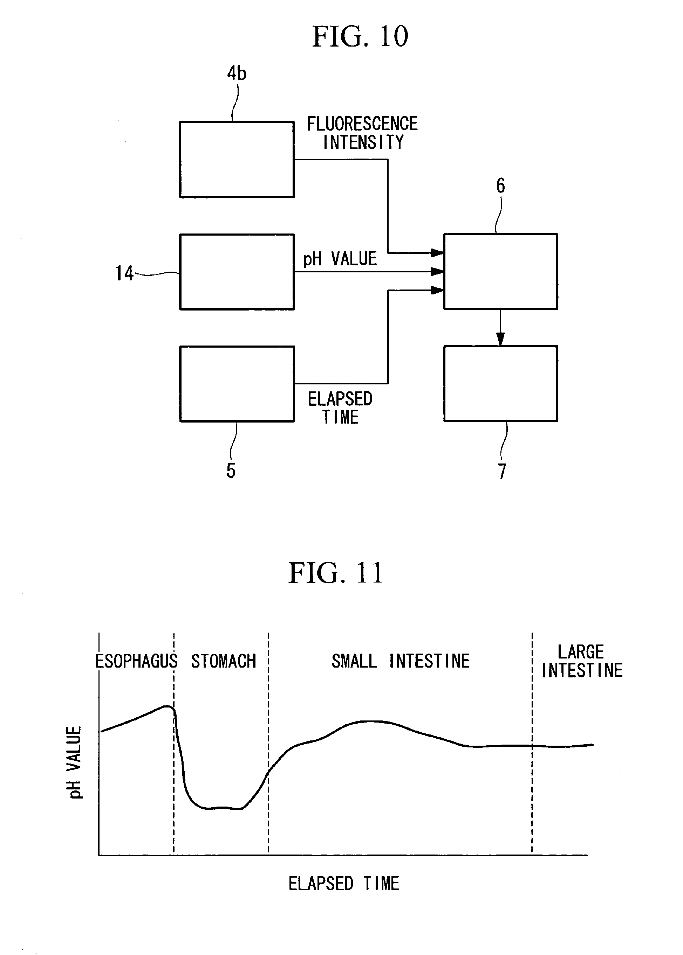

Third, as shown in Fig. 10, the examination sites corresponding to the fluorescence intensity information may be located on the basis of variations in pH value measured using a pH sensor 14. For example, as shown in Fig. 11, it can be determined on the basis of the output of the pH sensor 14 that the examination site has shifted to the stomach because the stomach is lower in pH value than other organs.
Again, because pH values and examination sites cannot be uniquely associated if the pH sensor 14 is used alone, it is necessary to use the elapsed time measured by the timer 5 in combination therewith, as shown in Fig. 10, or to store the acquired pH values in chronological order.

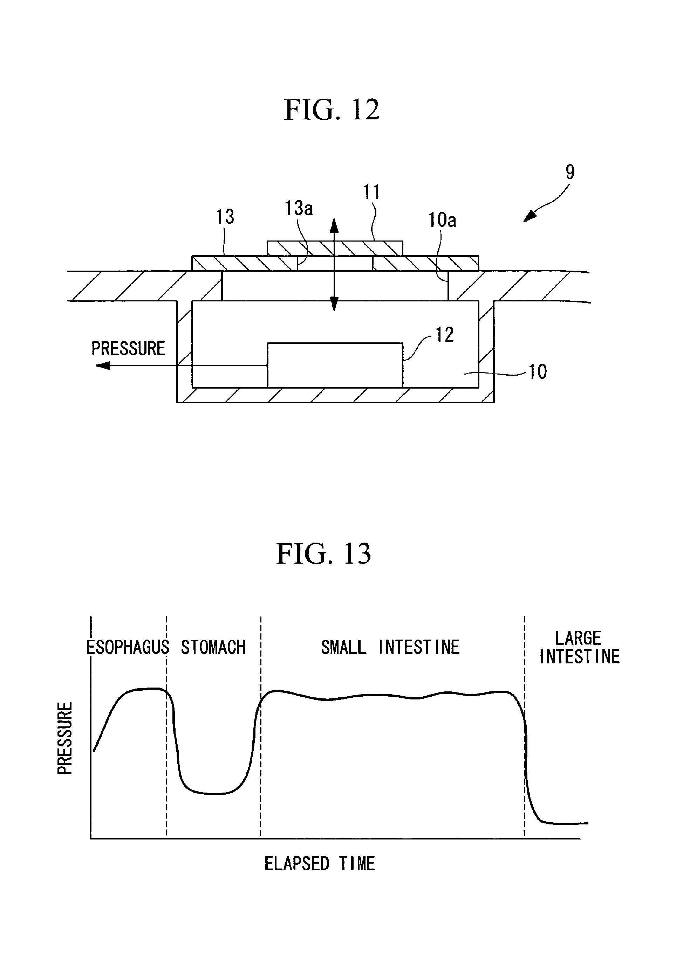

In addition, the timer 5, the pressure sensor 12, the enterobacterium sensor 9, and the pH sensor 14 may be used in a combination of two or more.
For example, as shown in Fig. 12, if the timer 5 (see Fig. 1.), the pressure sensor 12, and the enterobacterium sensor 9 are combined, an opening 13a provided in the elastic film 13 sealing off the opening 10a of the pressure chamber 10 may be sealed off with the azo polymer film 11.

By doing so, the azo polymer film 11 is maintained without rupturing at examination sites other than the large intestine, so that, as shown in Fig. 13, the elastic film 13 varies the internal pressure of the pressure chamber 10 through elastic deformation depending on the examination site, thus varying the pressure detected by the pressure sensor 12. On the other hand, if the capsule medical device 1 moves to the large intestine, the azo polymer film 11 is ruptured by an enzyme secreted by enterobacteria so that the internal pressure of the pressure chamber 10 drops. Thus, the examination site can be more precisely located on the basis of pressure variations.

In addition, for example, the timer 5, the enterobacterium sensor 9, and the pH sensor 14 may be combined.
By doing so, the stomach can be located on the basis of the pH values measured by the pH sensor 14, the large intestine can be located by the enterobacterium sensor 9, and the esophagus and the small intestine, lying therebefore and thereafter, can be more precisely located on the basis of elapsed time.
In particular, if fluorescence intensity is used as measurement information, it is advantageous in saving memory because the amount of information is small, although the examination site cannot be located because no image information is available. If fluorescence intensity is used as measurement information, therefore, an examination site locater can be used to achieve both locating of the examination site and memory saving, which is a particularly significant advantage.

In addition, whereas fluorescence intensity information is taken as an example of the measurement information for the examination site in this embodiment, two-dimensional image information may instead be acquired by replacing the photodetector 4b with a CCD. Because directly storing two-dimensional image information involves an enormous amount of data, the information-processing section 6 may extract luminance information and area information from the two-dimensional image information and associate the extracted luminance information and area information with elapsed time.

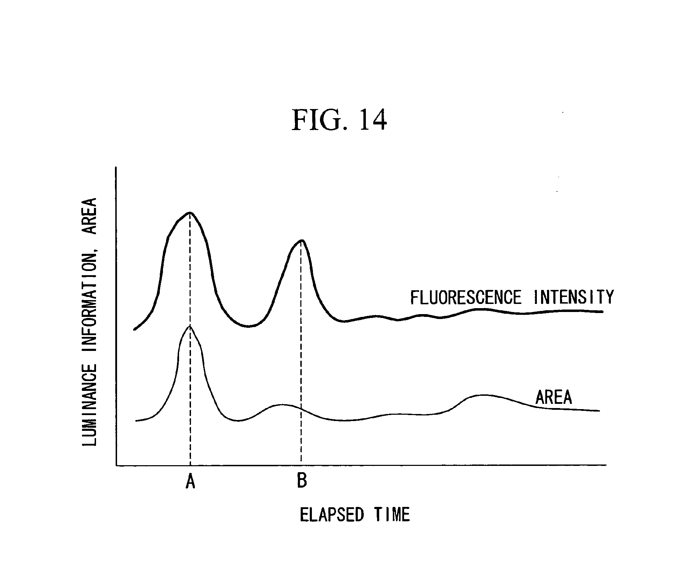

The luminance information extracted may be, for example, the maximum or average value of the fluorescence intensities of the individual pixels. In addition, the area information may be the area of a region where the fluorescence intensity is at or above a predetermined threshold (that is, the number of pixels having such fluorescence intensities).
By doing so, as shown in Fig. 14, it is possible to prevent acquisition, due to noise, of an image of an examination site having a relatively narrow area and a high fluorescence intensity (reference B in Fig. 14), thus more reliably locating an examination site that is possibly diseased (reference A in Fig. 14).

## Claims

1. A capsule medical device comprising:
a casing having a capsule shape;
a measurement-information acquiring section, disposed in the casing, for acquiring measurement information from an examination site positioned outside the casing;
a locating-information acquiring section for acquiring information for locating the examination site from which the measurement information has been acquired by the measurement-information acquiring section; and
a storage section for storing the measurement information acquired by the measurement-information acquiring section in association with the information for locating the examination site, acquired by the locating-information acquiring section.

2. The capsule medical device according to Claim 1, wherein the measurement-information acquiring section includes an image-acquisition device for acquiring image information by taking an image of the examination site outside the casing.

3. The capsule medical device according to Claim 2, wherein the measurement-information acquiring section calculates a maximum or average value of luminance values of individual pixels acquired by the image-acquisition device as the measurement information for the examination site.

4. The capsule medical device 1 according to Claim 2 or 3, wherein the measurement-information acquiring section calculates the number of pixels whose luminance values acquired by the image-acquisition device exceed a predetermined threshold as the measurement information for the examination site.

5. The capsule medical device according to Claim 1, wherein the measurement-information acquiring section includes a light detector for detecting brightness information at the examination site outside the casing.

6. The capsule medical device according to Claim 1, wherein the locating-information acquiring section is a timer for measuring examination time.

7. The capsule medical device according to Claim 1, wherein the locating-information acquiring section is a pressure sensor for detecting a pressure exerted on the casing.

8. The capsule medical device according to Claim 1, wherein the locating-information acquiring section is an enterobacterium sensor for detecting enterobacteria.

9. The capsule medical device according to Claim 1, wherein the locating-information acquiring section is a pH sensor for detecting a pH value around the casing.
